# EUROPEAN PATENT APPLICATION

(11) **EP 1 605 038 A1**
(43) Date of publication of application: **14.12.2005**
(21) Application number: 04718436.1
(22) Date of filing: 08.03.2004
(51) Int. Cl.: C12N 5/00, A61K 35/12, A61L 27/00

(54) **METHOD OF CELL CULTURING AND CELL SHEET**

(30) Priority: 18.03.2003 JP 2003073808
(71) Applicant: Honda, Hiroyuki, Nagoya-shi, Aichi 458-0818 (JP); Japan Tissue Engineering Co., Ltd., Gamagori-shi, Aichi 443-0022 (JP)
(72) Inventor: HONDA, Hiroyuki, Nagoya-shi, Aichi 458-0818 (JP); ITO, Akira, Nagoya-shi, Aichi 458-0827 (JP); KOBAYASHI, Takeshi, Nagoya-shi, Aicha 464-0096 (JP); UEDA, Minoru, Nagoya-shii, Aichi 461-0011 (JP); KAGAMI, Hideaki, Nagoya-shi, Aichi 462-0831 (JP); HATA, Ken-ichiro, Kariya-shi, Aichi 448-0802 (JP); TERASAKI, Hiroko, Nagoya-shi, Aichi 460-0012 (JP)
(74) Representative: Kuhnen & Wacker
(86) International application number: PCT/JP2004/002967
(87) International publication number: WO 2004/083412

(57) **Abstract**

Epidermal keratinocytes not incorporating magnetic particles therein and epidermal keratinocytes incorporating magnetic particles therein were seeded in a 24-well ultra-low-attachment plate, and cultured for one day after a neodymium magnet was placed outside the bottom of the well. As a result, control epidermal keratinocytes not incorporating magnetic particles therein did not adhere to the bottom of the well and did not form an undifferentiated cell sheet. On the contrary, epidermal keratinocytes incorporating magnetic particles therein, while being attracted to the bottom of the well by magnetic force, formed a multilayered cell sheet. This cell sheet could be easily recovered by removing the neodymium magnet placed outside the bottom of the well, then bringing a magnet having a hydrophilic film attached thereto close to the cell sheet from upside and to attract it via this hydrophilic film, and lifting it.

## Description

### TECHNICAL FIELD

The present invention relates to a cell culture method for culturing adhesion-dependent cells and a cell sheet obtained thereby.

### BACKGROUND ART

Recently, much attention has focused on technologies for culturing cells in vitro and producing cultured tissue to be used as tissue for transplantation or for tests. Such technologies originates with the study by Howard Green et al. who cultured human epidermal cells by using inactivated mouse 3T3 cells as feeder cells in the beginning of the 1980s. When epidermal cell sheet is produced by using this kind of technology, since sheet is formed by culturing epidermal cells attach to the bottom of a culture container, a step for detaching the epidermal cell sheet from the bottom of the culture container by treatment with an enzyme such as dispase is carried out. However, since the enzyme treatment degrades adhesion protein on the surface of cells, there is a concern about damage to cells.

In order to resolve this concern, various proposals have been made. For example, Japanese Patent Examined Publication No. H6-104061 discloses a technology of detaching and recovering grown cells from the surface of a scaffold by changing the environmental temperature without enzyme treatment. As a specific example, there is a description as follows. A surface of a polystyrene Petri dish was coveredwithN-isopropyl acrylamide polymer or N, N-diethylacrylamide, followed by polymerization by electron beam irradiation, and thereafter, bovine aorta vascular endothelial cells were incubated at 37°C in 5% CO₂ using Dulbecco's modified Eagle's medium (DMEM) supplemented with 20% fetal calf serum (FCS), fully grown, then cooled to 4°C and allowed to stand. Thus, attached cultured cells were detached without enzyme treatment. According to the description, this result could be achieved because the surface of the Petri dish was changed from hydrophobic to hydrophilic by decreasing the temperature from 37°C to 4°C.

The use of such a temperature responsive polymer is being recognized as one of the techniques relating to a method for detaching culture tissue. However, it is necessary for the development of technology to provide more options on methods for detaching culture tissue without carrying out treatment with an enzyme. Furthermore, in the methodusing a temperature responsive polymer, it is possible to construct multilayered tissue by laminating monolayer cell sheets, but it takes a time to laminate a monolayer sheet each by each.

### DISCLOSURE OF THE INVENTION

With the foregoing problems in mind, an object of the present invention is to provide a cell culture method capable of recovering cultured cells from a culture container without enzyme treatment. Another obj ect of the present invention is to provide a cell culture method capable of recovering cultured cells from a culture container without greatly changing the environmental temperature. A further object of the present invention is to provide a cell culture method capable of obtaining multilayered tissue in a simple way. A yet further object of the present invention is to provide a novel cell sheet.

In order to achieve at least one of the above-mentioned objects, the present invention employs the following technique. The present invention provides a cell culture method for culturing cells. The method includes: a magnetization step for allowing adhesion-dependent cells to hold magnetic particles, thereby magnetizing the cells; a seeding step for seeding the magnetized cells in a culture container having a cell non-adhesive bottom; an attracting step for attracting the magnetized cells to the bottom of the culture container by magnetic force after the seeding step; a culture step for culturing the magnetized cells, while being attracted to the bottom of the culture container by magnetic force, until they reach a predetermined state; and a releasing step for removing the magnetic force after the magnetized cells reach the predetermined state, thereby releasing the cells that reached the predetermined state from the bottom of the culture container.

In this cell culture method, the magnetized cells are seeded in the culture container having the cell non-adhesive bottom, the magnetized cells are then attracted to the bottom of the culture container by magnetic force and cultured in this state until they reach a predetermined state, and the magnetic force is removed after the cells reached the predetermined state, thereby releasing the cells that reached the predetermined state from the cell non-adhesive bottom. That it to say, since the adhesion-dependent cells are attracted to the bottom of the culture container by magnetic force and quasi-adheres thereto, when the magnetic force is removed, the cells can be easily released from the bottom of the culture container. Consequently, cultured cells can be recovered from the culture container without carrying out enzyme treatment. Furthermore, as compared with the method using a temperature responsive polymer, the cells thus cultured can be recovered from the culture container without greatly changing the environmental temperature.

Examples of the adhesion-dependent cells (which adhere to a culture surface directly or indirectly, expand the adhesion area and then divide, and which may also be referred to as anchorage-dependent cells) to be used in the magnetization step of the present invention may include various cells obtained from warm-blooded animals such as human, mouse, rat, guineapig, hamster, chicken, rabbit, pig, sheep, cow, horse, dog, cat, monkey, etc. Examples of the cell of such warm-blooded animals may include keratinocyte, splenocyte, neurocyte, gliacell, pancreatic β cell, mesangium cell, Langerhans cell, epidermal cell, epithelial cell (including corneal epithelial cell, oral mucosal epithelial cell, amniotic membrane epithelial cell, retinal pigment epithelial cell, etc.), endothelial cell, fibroblast, fibrous cell, muscle cell, adipocyte, synoviocyte, chondrocyte, osteocyte, osteoblast, osteoclast, mammary glandular cell, hepatocyte or interstitial cell, or precursor cell thereof, and further stem cell such as embryonic stem cell (ESC), mesenchymal stem cell (MSC), etc., and adhesion-dependent cancer cell. Furthermore, as the cell non-adhesive bottom, any bottoms may be employed as long as they are bottoms to which adhesion-dependent cells do not adhere or do not easily adhere. Examples of such bottoms may include a bottom of a culture container (which may also include membrane) made of such materials as polystyrene, polypropylene, fluororesin, polytetrafluoroethylene (PTFE), polycarbonate, polyester, etc., a bottom of culture container (which may also include membrane) coated with agarose, agar, gelatin, collagen, fibrin, etc., a positively charged bottom of a culture container, and the like. Examples of the culture container having a cell non-adhesive bottom may include an ultra-low-attachment plate (trade name) from Corning, and the like. Note here that the bottom to which cells do not easily adhere means a bottom having adhesiveness to such an extent that when magnetic force is removed, magnetized cells can be separated from the bottom by lightly shaking the culture container.

The magnetic particles used in the magnetization step of the present invention is not particularly limited, and any particles may be employed as long as they can be held by the cells and thereby provide the cells with magnetic property. Examples of such magnetic particles may include magnetic particles constituting a magnetic particle cationic liposome (MPCL) in which magnetic particles such as magnetite are enclosed in a liposome, an antibody-immobilized magnetoliposome (AML) in which magnetic particles are enclosed in an antibody-immobilized liposome; magnetic micro-beads in MACS (Magnetic Cell Sorting and Separation of Biomolecules) produced by Daiichi Pure Chemicals; magnetic nanoparticles (trade name: EasySep) produced by VERITAS, and the like. Among these magnetic particles, magnetic particles containing liposomes such as MPCL and AML are preferable because they are taken up by cells by the presence of liposomes, a single cell can take up a large number of magnetic particles, and the cells can easily have a magnetic property to such an extent that the cells can be attracted to the bottom of the culture container by magnetic force.

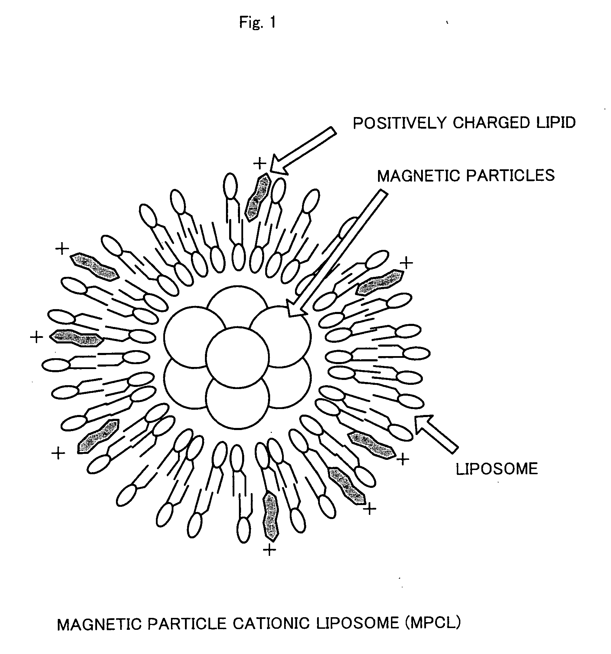

As shown in Fig. 1 as one example, the MPCL has a structure in which magnetic particles such as magnetite are enclosed in a liposome and the liposome is provided with positively charged lipid. Since many cells are negatively charged, they are easily coupled to positively charged MPCLs. Since MPCLs have liposomes, they are easily taken up by cells. Therefore, the present invention, when MPCLs are employed as the magnetic particle, can be applied for culturing various cells. MPCLs may be prepared with reference to the method for producing magnetite cationic liposome (MCL) described in, for example, Jpn. J. Cancer Res. Vol. 87 (1996), p. 1179-1183. When MPCLs are employed in the magnetization step, it is preferable that 1-150 pg/cell, particularly 20-150 pg/cell of MPCLs are used as the magnetic particles. It is preferable that at 0.5-8 hours, particularly 3-5 hours after cells to be cultured and MPCLs start to come into contact with each other in the magnetization step, a next step is carried out.

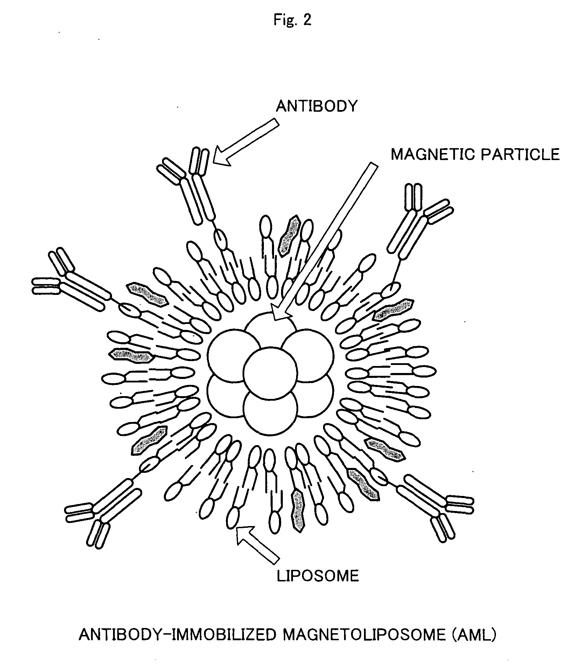

As shown in Fig. 2 as one example, AML has a structure in which magnetic particles such as magnetite are enclosed in a liposome and the liposome is provided with antibodies. As the antibody, an antibody with a property of specifically binding to certain cells to be cultured is selected. Cells having a site specifically binding to the antibody are easily bound to the antibody in the AML. Furthermore, since AMLs have liposomes, they are easily taken up by cells. AMLs may be prepared with reference to the producing method described in, for example, J. Chem. Eng. Jpn. Vol. 34 (2001), p. 66-72. When AMLs are employed in the magnetization step, it is preferable that 1-150 pg/cell, particularly 20-150 pg/cell of AMLs are used as the magnetic particles. It is preferable that at 0.5-8 hours, particularly 3-5 hours after cells to be cultured and AMLs start to come into contact with each other in the magnetization step, a next step is carried out.

In the seeding step of the present invention, the seeding density, etc. may be appropriately set depending upon the kind of cells, the size of intended culture tissue, and the like, but is generally set in the range from 1×10³ cells/cm² to 1×10⁶ cells/cm².

In the attracting step of the present invention, magnetic force for attracting the magnetized cells to the bottom of the culturecontainermaybeapplied. For example, when the magnetized cells are attracted to the bottom of the culture container with a magnet placed outside the bottom of the culture container, the magnetic force is appropriately determined based on the kind of magnetic particles, the amount of magnetic particles taken up by cells, materials and thickness of the bottom of the culture container, and the like.

In the culture step of the present invention, the kind of liquid culture medium may be appropriately selected depending upon the kind of cells to be cultured. For example, well-known DMEM, α-MEM, M199 medium, and the like, may be selected. Furthermore, additive factor such as growth factor represented by EGF or FGF may be appropriately added. In this culture step, cells are cultured until they reach a predetermined state. The predetermined state may be appropriately selected in accordance with the purpose. For example, cells may be cultured until the cultured cells form a cell sheet. At this time, the cell sheet may be a monolayer cell sheet or a multilayered cell sheet. Alternatively, when used for subculture, a dispersion state for each cell may be selected in consideration of ease in subculture operation.

In the releasing step of the present invention, in removing magnetic force, the magnetic force may be reduced to such an extent that the magnetized cells are not attracted to the bottom of the culture container. For example, in the case where the magnetized cells were attracted to the bottom of the culture container by a magnet placed outside the bottom of the culture container, the distance between the magnet and the bottom of the culture container is determined based on the kind of magnetic particles, the amount of magnetic particles takenupby cells, the materials and thickness of the bottom of the culture container, and the like.

The cell culture method of the present invention may include a recovering step for recovering the cells that reached a predetermined state by magnetic force after the releasing step. In this recovering step, the cells may be recovered by putting a suspending support film in a culture container, allowing the cells that reached the predetermined state to be attracted to the support film by magnetic force, and then lifting the suspending support film. Herein, the suspending support film is not particularly limited, and any one may be employed as long as it can suspend the cells that reached the predetermined state substantially as it is. Examples of such a suspending support film may include knit fabric, woven fabric, non-woven fabric, paper, resin sheet, and the like. For example, sterile gauze, sterile Japanese paper, sterile filter paper and sterile non-woven fabric, a hydrophilic film (including a film the surface of which was treated to have hydrophilic property) such as a PVDF film (polyvinylidene fluoride film), a PTFE film (polytetrafluoroethylene film), etc., a sheet-like material of macromolecular materials with flexibility such as silicone rubber, a biodegradable polymer such as polyglycolic acid, polylactic acid, etc., and hydrogel such as agar medium, collagen gel, gelatin gel, etc., and the like may be used as a suspending support film. As the magnetic force, magnetic force of an electromagnet capable of controlling the magnetization and demagnetization by being energized and de-energized may be used. This is convenient because not only an operation of recovering cells that reached a predetermined state can be easily automated but also operations of delivering and packaging cells can be easily automated.

Since the cell sheet produced by the cell culture method of the present invention is cultured in a state in which it does not adhere directly to the culture container but is attracted to the culture container by magnetic force, when a plurality of cell layers are laminated, all or many of the cell layers are undifferentiated cell layers. Such a cell sheet that is rich in undifferentiated cell layers has never been known to date. When such a cell sheet is transplanted into a wound portion, a high wound healing effect can be expected.

### BRIEF DESCRIPTION OF THE DRAWINGS

Fig. 1 is a schematic view showing one example of a magnetic particle cationic liposome (MPCL).
Fig. 2 is a schematic view showing one example of an antibody-immobilized magnetoliposome (AML).
Fig. 3 is a photograph showing a cross-section of a cell sheet.
Fig. 4 is a graph showing the relation ship between incubation time and magnetite uptake.
Fig. 5 is a graph showing the relation ship between incubation time and viable cell number.
Fig. 6 is a photograph showing a cross-section of a hematoxylin and eosin-stained RPE (retinal pigment epithelium) cell sheet.
Fig. 7 is a view to illustrate a state in which a RPE cell sheet is transferred. (a) shows a state before the cell sheet is attracted to a wire; (b) shows a state at the time the cell sheet is attracted to the wire; and (c) shows a state when the cell sheet is released from the wire.

### BEST MODE FOR CARRYING OUT THE INVENTION

### [Example 1]

In Example 1, the case in which epidermal cells (keratinocytes) are cultured to produce a cultured epidermis sheet will be described.

### (1) Cells to be used

Normal human epidermal keratinocytes commercially available for studies from Kurabo were used. As a culture medium for epidermal keratinocytes, for a growth medium, a serum free medium, HuMedia-KG2 (Kurabo) was used, and for a differentiation inducing medium, HuMedia-KG2 supplemented with calcium chloride to have the total calcium concentration had been adjusted to 1.0 mM was used.

### (2) Preparation of magnetite cationic liposomes (MCLs)

MCLs were prepared based on the method described in Jpn. J. CancerRes. Vol. 87 (1996), p. 1179-1183. Specifically, firstly, liposome membrane containing three kinds of phospholipids, that is, N-(α-trimethylammonioacetyl)-didodecyl-D-glutamate chloride (Sogo Pharmaceutical), dilauroylphosphatidyl-choline (Sigma Chemicals), and dioleoylphosphatidyl-ethanolamine (Sigma Chemicals) in a 1 : 2 : 2 molar ratio was formed, subsequently 1 mL of colloidal magnetite (amount of magnetite: 20 mg; Toda Kogyo) was added, and the well-known vortex method was carried out to thus prepare MCLs. The magnetite concentration measured using the potassium thiocyanate method was 18 mg/ml.

### (3) MCL uptake by epidermal keratinocytes

Epidermal keratinocytes were suspended in a growth medium to prepare a cell suspension, which was seeded in a culture dish (Asahi Techno Glass) at 1×10⁴ cells/cm² and allowed to stand until cells adhere to the bottom of the culture dish. Thereafter, a growth medium was added, and incubation was carried out in a CO₂ incubator. Incubation was carried out at 37°C under a humidified air atmosphere containing 5% CO₂. At the first replacement of culture media, a culture medium of epidermal keratinocytes in logarithmic proliferation phase, which had been conditioned, were replaced with another culture medium. At this time, for a fresh growth medium for replacement, a culture medium added with MCLs was used. The MCLs were added so that the MCL concentration became 50 pg/cell as magnetite for respect to the number of epidermal keratinocytes. Then, epidermal keratinocytes were cultured in the MCL-containing growth medium to thus allow the epidermal keratinocytes to take up MCLs.

### (4) Cell culture method using magnet

After epidermal keratinocytes incorporating MCLs therein became substantially confluent state, the cells were detached from the bottom of the culture dish by trypsinization and seeded in a24-wellultra-low-attachment plate having a cell non-adhesive bottom (Corning). The cells were seeded at 2×10⁶ cells/well. In the meanwhile, as a control, epidermal keratinocytes not incorporating MCLs therein were seeded in a 24-well ultra-low-attachment plate (Corning) at 2×10⁶ cells/well.

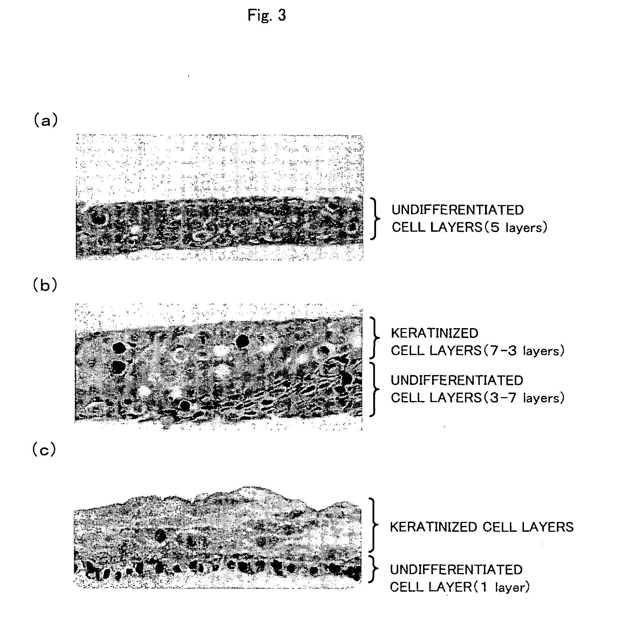

After seeding, a neodymium magnet (outer diameter: 3.0 cmφ, surfacemagnetic flux density: 0.45T) was placed outside the bottom of each well, followed by culturing for one day. Then, it was observed whether or not the epidermal keratinocytes form an undifferentiated cell sheet. Furthermore, the culture medium was replaced with a differentiation inducing medium and one-day culturingwas then carried out with neodymium magnet placed. Then, it was observed whether or not the epidermal keratinocytes were differentiated to form a multilayered cell sheet. As a result, control epidermal keratinocytes not incorporating MCLs therein did not attach to the bottom of the well and did not form an undifferentiated cell sheet. On the contrary, epidermal keratinocytes incorporating MCLs therein formed a five-layered undifferentiated cell sheet (see Fig. 3(a)). This undifferentiated cell sheet could be easily recovered by removing the neodymium magnet placed outside the bottom of the well, then bringing a stick-shaped alnico magnet (outer diameter: 1.0 cmφ, remaining magnetic flux density: 1.27T) having a hydrophilic-treated PVDF film (polyvinylidene fluoride film) attached at the tip thereof close to the surface of the culture solution from the upper side so as to allow the cell sheet to float up to the surface of the culture solution by magnetic force, attracting the cell sheet to the magnet via the PVDF film, and lifting it as it is. Furthermore, when the undifferentiated cell sheet was cultured in a differentiation inducing medium for one more day, a 10-layered cell sheet in which a part of the undifferentiated cells had been differentiated was formed (see Fig. 3(b)). This multilayered cell sheet shrunk due to differentiation, and when the neodymium magnet placed outside the bottom of the well was removed, the cell sheet floated in the culture medium and was easily released from the bottom of the well. Furthermore, this multilayered cell sheet was strong enough so that it could be handled with forceps.

By the way, Fig. 3(c) is a cross-sectional view showing a conventional cell sheet obtained by the culture method by Green et al. and can be found in publication "Jintai Saisei (Regeneration of Human Body)" (Takashi Tachibana, CHUOKORON-SHINSHA, INC. June 10, 2000: p. 229). It is thought that according to this culture method by Green et al., cells adhere to the bottom of the culture container, thereby differentiation occurs spontaneously, and a multilayered cell sheet includingmanykeratinized layers as shown in Fig. 3(C) was obtained. On the contrary, it is thought that in the above-mentioned Example, cells were cultured in a state in which the cells were not allowed to directly adhere to the culture container but attracted to the culture container by magnetic force and therefore the cells in undifferentiated state were multilayered. Incidentally, in Fig. 3(a), almost all the cell layers are undifferentiated cell layers. Also in Fig. 3 (b), as compared with Fig. 3(c), a larger number of cell layers are undifferentiated cell layers.

### [Example 2]

In Example 2, the case in which retinal pigment epithelial cells (hereinafter referred to as RPE cells) are cultured to form a cell sheet will be described.

### (1) Cells to be used, etc.

As normal human RPE cells, ARPE-19 cells provided by ATCC (American Type Culture Collection) were used. The cells were cultured at 37°C under a humidified air atmosphere containing 5% CO₂. As a culture medium, DMEM/HAM's F12 supplemented with 10% fetal calf serum and antibiotics (100 U/ml penicillin and 0.1 mg/ml streptomycin) was used.

### (2) Preparation of MCLs

MCLs were prepared by the same method as mentioned in (2) of Example 1.

### (3) MCL uptake by RPE cells

ARPE-19 cells were suspended in the culturemedium to prepare a cell suspension, which was seeded in a culture dish (Asahi Techno Glass) at 6×10⁵ cells/cm². After 24 hours of incubation, the culture medium was replaced with another culture medium containing MCLs (magnetite concentration: 25 pg/cell or 50 pg/cell), and the cells were further incubated. To analyze themagnetiteuptake, the cells were sampled periodically. The iron concentration was measured using the potassium thiocyanate method and viable cell number was measured by the dye-exclusion method using tripanblue, respectively.

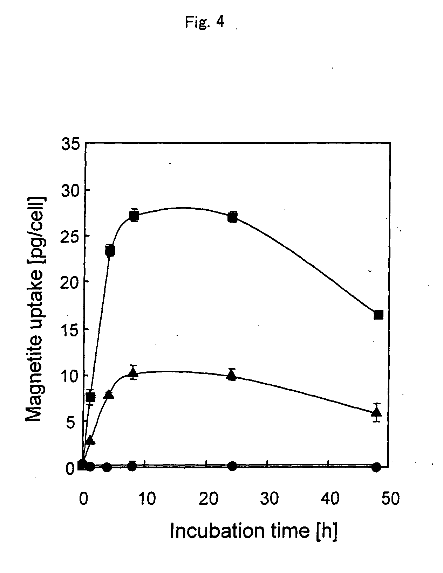

The uptake of magnetite by ARPE-19 cells began rapidly. At 8 hours after the start of the incubation in a culture medium containing MCLs, the magnetite concentration in the cells reached maximum values, 10 and 27 pg/cell in the culture media having the magnetite concentrations of 25 and 50 pg/cell, respectively (see Fig. 4). Thereafter, the magnetite concentration of the cells was diluted due to the cell proliferation, and at 48 hours after the start of the incubation, the concentration was slightly diluted.

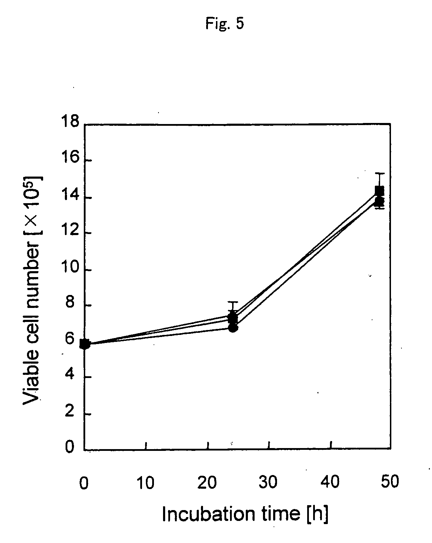

Furthermore, the proliferation of ARPE-19 cells in a culture medium containing MCLs (magnetite concentration: 25 pg/cell, 50 pg/cell) was compared with the proliferation of ARPE-19 cells in a culture medium without MCLs to investigate the toxicity of MCLs for ARPE-19 cells. However, MCLs did not inhibit the proliferation of ARPE-19 cells at any of concentrations (see Fig. 5). Consequently, in the following experiments, for allowing ARPE-19 cells to take up magnetite, a culture medium having a magnetite concentration of 50 pg/cell was used.

### (4) Cell culture method by using magnet

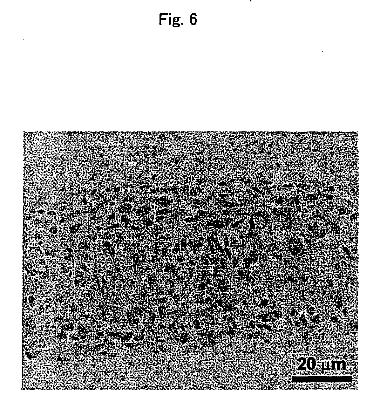

A multilayered cell sheet with the size of 4 mm² or smaller was produced by culturing ARPE-19 cells while accumulating them by magnetic force. Specifically, a cell sheet was produced by the following procedure and the resultant cell sheet was evaluated. That is to say, at 4 hours after the addition of MCLs, 3×10⁴ cells were seeded into a 2.4 mm-diameter cloning ring (height: 10 cm; inside area: 4 mm²; Asahi Techno Glass) placed on the center of a 24-well ultra-low-attachment plate (Corning). Note here that the cells were seeded at a cell density of 8×10³ cells/mm², which corresponds to the density of 10-fold cell layers of confluent state. The surface of the plate is a hydrophilic hydrogel layer. Then, a cylindrical neodymium magnet (diameter: 22 mm; height: 10 mm; 4000 Gauss) was placed on the center on the reverse side of the surface of the ultra-low-attachment plate to provide magnetic force vertical to the bottom of the plate. When the cells were cultured for one day in this state, a sheet structure of 1 mm² was formed. In order to confirm whether the obtained RPE cell sheet was multilayered, the cross-section thereof was observed. It was shown that the ARPE-19 cells containing MCLs formed a 15-layered sheet in a thickness of 60 µm (see Fig. 6). Furthermore, hematoxylin and eosin staining revealed that no remarkable necrotic areas were observed in the cell sheet.

Note here that when ARPE-19 cells without containing MCLs were used or when ARPE-19 cells incorporating MCLs therein were cultured without using a magnet placed under the plate, a cell sheet was not formed and cells were dispersed by removing a cloning ring.

### (5) Recovery and delivery by using magnet

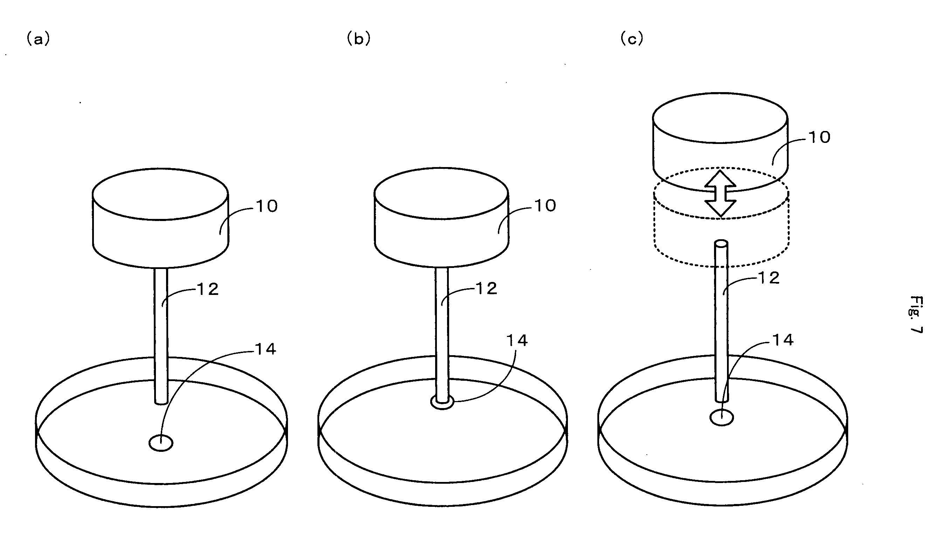

At one day after the start of culture, the magnet placed on the reverse side of the 24-well ultra-low-attachment plate was removed. Then, the RPE cell sheet was released from the bottom of the well. Subsequently, as a device for delivery, as shown in Fig. 7, an iron wire 12 (diameter: 2 mm; length: 40 mm), which was attracted to the center of the cylindrical neodymium magnet 10 (diameter: 30 mm; height: 15 mm; 4000 Gauss) by magnetic force, was prepared. When the tip of this wire 12 was positioned at the surface of the culture medium, the RPE cell sheet 14 floated up to the surface of the culture medium without disruption by magnetic force and was attracted to the tip of the wire 12. Note here that the magnetic flux density at the tip of the wire 12 was 1100 Gauss. Then, the RPE cell sheet 14 attracted to this wire 12 was transferred to a 100 mm-diameter culture dish (Asahi TechnoGlass) containing 10 ml of culturemedium. After the magnet 10 was removed from the wire 12 and the wire 12 was tapped gently, the RPE cell sheet was released from the wire and sank onto the surface of the culture medium. We judged from this result that this device for delivery enabled the recovery and delivery of a RPE cell sheet. Subsequently, the RPE cell sheet was incubated in the culture dish. After one-day incubation, the RPE cell sheet attached to the culture dish. Subsequently, the RPE cell sheet was further incubated. At day 16, outgrowth cells from the cell sheet were proliferated actively.

The present invention is not limited to the above- mentioned Examples. It is natural that various modifications which are within the scope in the art of the present invention are included in the present invention.

### INDUSTRIAL APPLICABILITY

The present invention is applicable in cell culture technologies for culturing cells invitro and canbe used inmedical equipment industry using prosthesis for each part of the body, for example, a cell sheet as medical device.

## Claims

1. A cell culture method for culturing cells, the method comprising:
a magnetization step for allowing adhesion-dependent cells to hold magnetic particles, thereby magnetizing the cells;
a seeding step for seeding the magnetized cells in a culture container having a cell non-adhesive bottom;
an attracting step for attracting the magnetized cells to the bottom of the culture container by magnetic force after the seeding step;
a culture step for culturing the magnetized cells, while being attracted to the bottom of the culture container by magnetic force, until they reach a predetermined state; and
a releasing step for removing the magnetic force after the magnetized cells reach the predetermined state, thereby releasing the cells that reached the predetermined state from the bottom of the culture container.

2. The cell culture method according to claim 1, wherein the magnetic particles are enclosed in a liposome to form a magnetic particle cationic liposome (MPCL), and in the magnetization step, the cells are magnetized by allowing the cells to take up the MPCL.

3. The cell culture method according to claim 1, wherein the magnetic particles are enclosed in an antibody-immobilized liposome to form an antibody-immobilized magnetoliposome (AML), and in the magnetization step, the cells are magnetized by binding the cells to the AML in which antibodies that are specifically bound to the cells are immobilized.

4. The cell culture method according to any of claims 1 to 3, comprising:
a recovering step for recovering the cells that reached the predetermined state by magnetic force after the releasing step.

5. The cell culture method according to claim 4, wherein in the recovering step, the cells are recovered by putting a suspending support film ina culture container, allowing the cells that reached a predetermined state to be attracted to the support film by magnetic force, and then lifting the suspending support film.

6. The cell culture method according to claim 5, wherein in the recovering step, magnetic force of an electromagnet capable of controlling the magnetization and demagnetization by being energized and de-energized is used as the magnetic force.

7. The cell culture method according to any of claims 1 to 6, wherein the adhesion-dependent cell is an epidermal cell or a retinal pigment epithelial cell.

8. The cell culture method according to any of claims 1 to 7, wherein in the culture step, the magnetized cells are cultured, while being attracted to the bottom of the culture container by magnetic force, until the cultured cells form a cell sheet.

9. The cell culture method according to claim 8, wherein in the culture step, the magnetized cells are cultured, while being attracted to the bottom of the culture container by magnetic force, until the cultured cells form a multilayered cell sheet.

10. A cell sheet produced by the cell culture method according to claim 8 or 9.

11. A cell sheet comprising a plurality of cell layers that are laminated, wherein almost all the cell layers are undifferentiated cell layers.

12. A cell sheet comprising adhesion-dependent cells holding magnetic particles.
